# EUROPÄISCHE PATENTSCHRIFT

(11) **EP 0 565 961 B1**
(45) Veröffentlichungstag und Bekanntmachung des Hinweises auf die Patenterteilung: **07.07.1999**
(21) Anmeldenummer: 93105424.1
(22) Anmeldetag: 01.04.1993
(51) Int. Cl.: C07D 249/08, A01N 43/653

(54) **Ethyl-triazolyl-Derivate**
Ethyl-triazolyl derivatives
Dérivés d'éthyl-triazolyle

(30) Priorität: 14.04.1992 DE 4212424; 04.12.1992 DE 4240867
(43) Veröffentlichungstag der Anmeldung: 20.10.1993
(73) Patentinhaber: BAYER AG, 51368 Leverkusen (DE)
(72) Erfinder: Scherkenbeck, Jürgen, Dr., W-5090 Leverkusen 3 (DE); Lindemann, Michael, Dr., W-5249 Hamm (DE); Dutzmann, Stefan, Dr., W-4010 Hilden (DE); Dehne, Heinz-Wilhelm, Dr., W-4019 Monheim (DE)

(56) Entgegenhaltungen:
- EP-A- 0 090 269
- EP-A- 0 097 425
- EP-A- 0 110 570
- EP-A- 0 237 917
- EP-A- 0 297 345
- EP-A- 0 353 558

## Beschreibung

Die vorliegende Erfindung betrifft neue Ethyl-triazolyl-Derivate, ein Verfahren zu deren Herstellung und deren Verwendung als Fungizide.

Es ist bereits bekannt geworden, daß bestimmte Halogenalkyl-triazolyl-Derivate fungizide Eigenschaften besitzen (vergleiche EP-A 0 097 425). So lassen sich z.B. 4-(2,4-Dichlor-phenyl)-1,2-dibrom-4-hydroxy-5-(1,2,4-triazol-1-yl)-pent-1-en und 4-(2,4-Dichlor-phenyl)-1,2-dichlor-4-hydroxy-5-(1,2,4-triazol-1-yl)-pent-1-en zur Bekämpfung von Pilzen einsetzen. Die Wirkung dieser Stoffe ist gut, läßt aber bei niedrigen Aufwandmengen in manchen Fällen zu wünschen übrig.

Weiterhin sind aus der EP - A 0 353 558, der EP - A 0 237 917 und der EP - A 0 297 345 zahlreiche 2 - Hydroxyethyl-azolyl-Derivate mit fungiziden Eigenschaften bekannt.

Schließlich werden in der EP - A 0 110 570 und der EP - A 0 090 269 fungizid wirksame Azolyl-Derivate offenbart, in denen am Kohlenstoffatom in 2-Position zum Azolylrest entweder eine Doppelbindung oder ein Halogenatom vorhanden ist. Keine der Substanzen enthält jedoch einen Cyclopropyl-Rest.

Es wurden nun neue Ethyl-triazolyl-Derivate der Formel 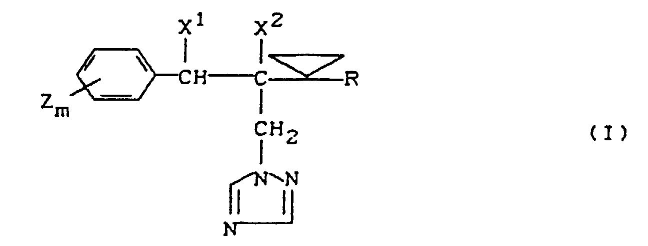
- R: für Fluor oder Chlor steht,
- X¹: für Fluor, Chlor oder Brom steht,
- X²: für Fluor, Chlor oder Brom steht,
- Z: für Fluor oder Chlor steht und
- m: für die Zahlen 0, 1 oder 2 steht,
sowie deren Säureadditions-Salze und Metallsalz-Komplexe gefunden.

Die Verbindungen der Formel (I) enthalten mindestens zwei asymmetrisch substituierte Kohlenstoffatome und können deshalb in optischen Isomerenformen anfallen. Die vorliegende Erfindung betrifft sowohl die Isomerengemische als auch die einzelnen Isomeren.

Weiterhin wurde gefunden, daß man Ethyl-triazolyl-Derivate der Formel (I) sowie deren Säureadditions-Salze und Metallsalz-Komplexe erhalt, wenn man Ethyl-triazolyl-Derivate der Formel 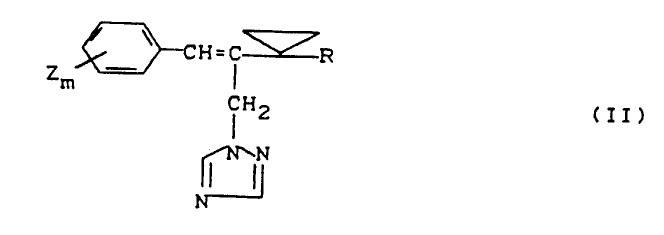 in welcher
R, Z und m die oben angegebenen Bedeutungen haben,
mit Halogenen der Formel

X¹-X² (III)

in welcher
X¹ und X² die oben angegebenen Bedeutungen haben,
oder mit Verbindungen, die Halogene der Formel (III) liefern, in Gegenwart eines Verdünnungsmittels und gegebenenfalls unter Bestrahlung umsetzt,
und gegebenenfalls an die so erhaltenen Verbindungen der Formel (I) eine Säure oder ein Metallsalz addiert.

Außerdem wurden neue Ethyl-triazolyl-Derivate der Formel 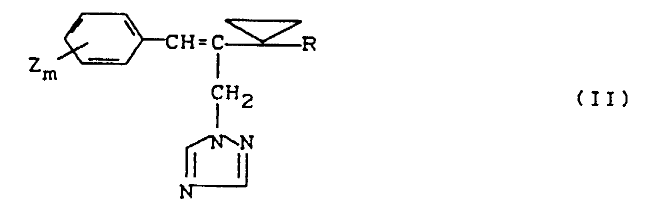 in welcher
- R: für Fluor oder Chlor steht,
- Z: für Fluor oder Chlor steht und
- m: für die Zahlen 0, 1 oder 2 steht,
sowie deren Säureadditions-Salze und Metallsalz-Komplexe gefunden.

Die Verbindungen der Formel (II) können in Abhängigkeit von der Stellung der Substituenten an der Doppelbindung in Form von geometrischen Isomeren vorliegen. Befinden sich der Phenyl-Rest und der Cyclopropyl-Rest auf entgegengesetzten Seiten der Doppelbindung, so handelt es sich um E-Isomere. Stehen der Phenyl-Rest und der Cyclopropyl-Rest auf der gleichen Seite der Doppelbindung, so handelt es sich um Z-Isomere. Die vorliegende Erfindung betrifft sowohl die Isomerengemische als auch die einzelnen Isomeren.

Ferner wurde gefunden, daß man Ethyl-triazolyl-Derivate der Formel (II) sowie deren Säureadditions-Salze und Metallsalz-Komplexe erhält, wenn man Azolylmethyl-ketone der Formel 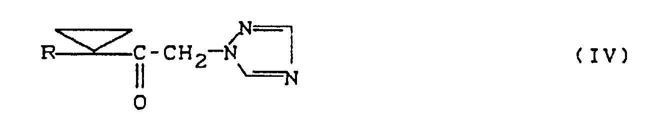 in welcher
- R: die oben angegebene Bedeutung hat,
mit Phosphonium-Salzen der Formel 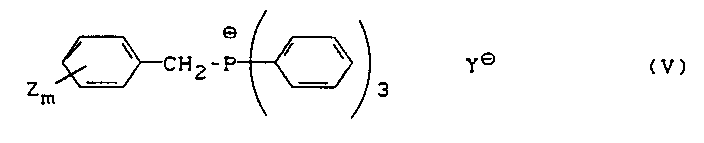 in welcher
- Z und m: die oben angegebenen Bedeutungen haben und
- Y: für Chlor oder Brom steht,
in Gegenwart einer Base und in Gegenwart eines Verdünnungsmittels umsetzt,
und gegebenenfalls an die so erhaltenen Verbindungen der Formel (II) eine Säure oder ein Metallsalz addiert.

Schließlich wurde gefunden, daß die neuen Ethyl-triazolyl-Derivate der Formeln (I) und (II) sowie deren Säureadditions-Salze und Metallsalz-Komplexe sehr gute fungizide Eigenschaften besitzen.

Überraschenderweise zeigen die erfindungsgemäßen Stoffe eine wesentlich bessere fungizide Wirksamkeit als die konstitutionell ähnlichsten, vorbekannten Verbindungen gleicher Wirkungsrichtung.

Erfindungsgemäße Stoffe sind auch Additionsprodukte aus Säuren und denjenigen Ethyl-triazolyl-Derivaten der Formel (I), in denen R, X¹, X², Z und m die oben genannten Bedeutungen haben, bzw. denjenigen Ethyl-triazolyl-Derivaten der Formel (II), in denen R, Z und m die oben genannten Bedeutungen haben.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, und außerdem auch Saccharin und Thiosaccharin.

Erfindungsgemäße Stoffe sind außerdem Additionsprodukte aus Salzen von Metallen der II, bis IV. Haupt- und der I, und II. sowie IV. bis VIII, Nebengruppe des Periodensystems der Elemente und Ethyl-triazolyl-Derivaten der Formel (I), in denen R, X¹, X², Z und m die oben genannten Bedeutungen haben, bzw. denjenigen Ethyl-triazolyl-Derivaten der Formel (II), in denen R, Z und m die oben genannten Bedeutungen haben.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle 1 aufgeführten Ethyl-triazolyl-Derivate genannt.

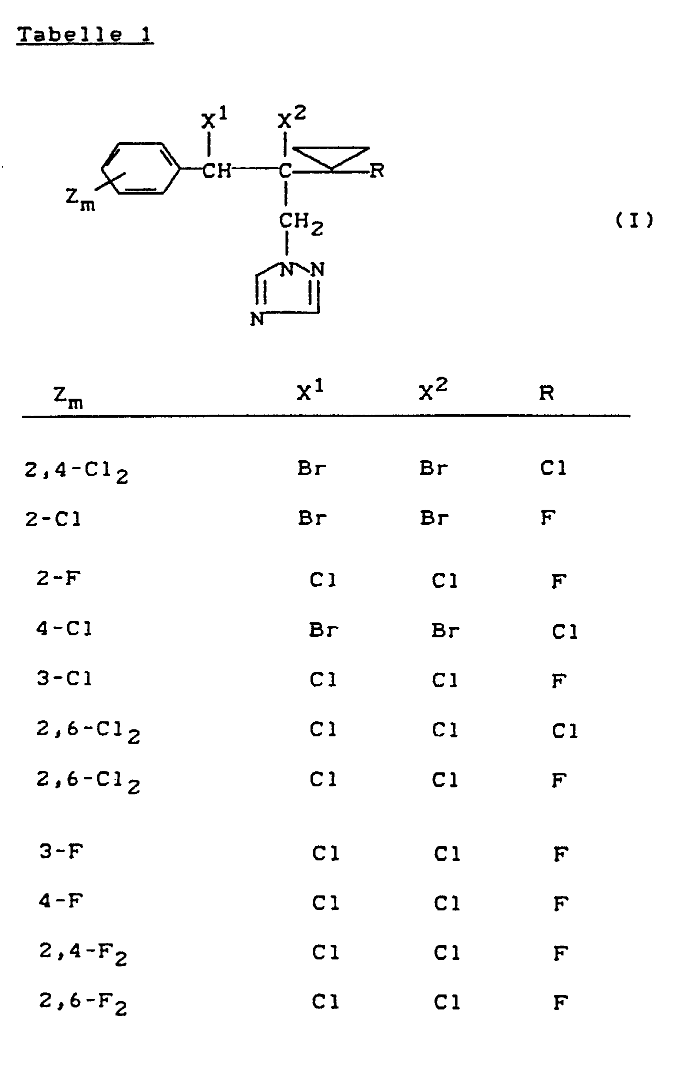

Verwendet man 2-(1-Chlorcyclopropyl)-1-(1,2,4-triazol-1-yl)-3-(2-chlorphenyl)-2-propen als Ausgangsstoff und Chlorgas als Reaktionskomponente, so kann der Verlauf des erfindungsgemäßen Verfahrens zur Herstellung von Verbindungen der Formel (I) durch das folgende Formelschema veranschaulicht werden: 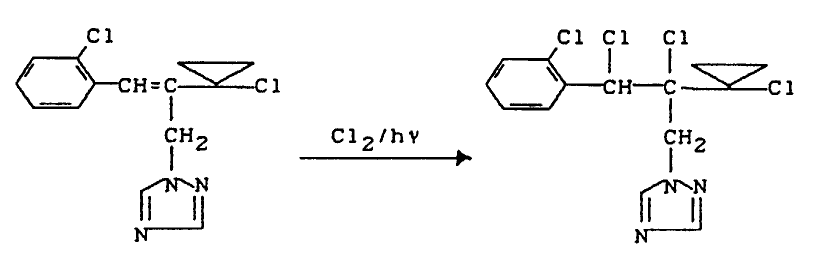 Verwendet man 1-Chlor-cyclopropyl-(1,2,4-triazol-1-yl)-methylketon und 2-Chlorbenzyl-triphenylphosphoniumchlorid als Ausgangsstoffe und Kalium-tert.-butylat als Base, so kann der Verlauf des erfindungsgemäßen Verfahrens zur Herstellung von Verbindungen der Formel (II) durch das folgende Formelschema veranschaulicht werden:

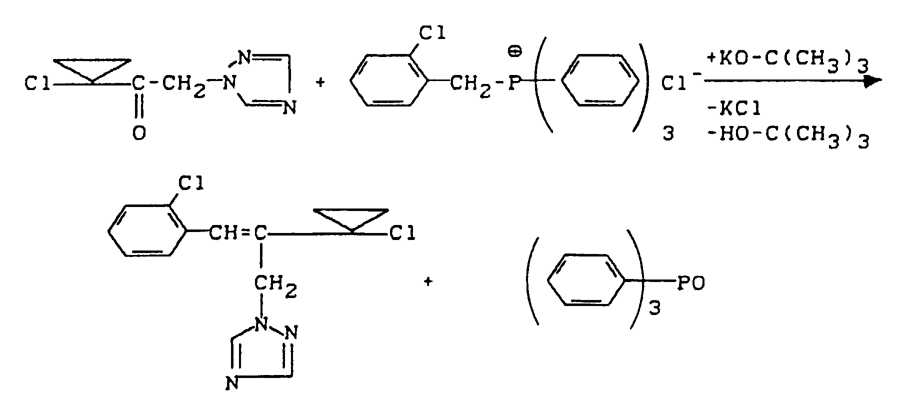

Die bei der Durchführung des erfindungsgemäßen Verfahrens zur Herstellung von Verbindungen der Formel (I) als Ausgangsstoffe benötigten Ethyl-triazolyl-Derivate der Formel (II) sind, wie bereits oben erwähnt, neue erfindungsgemäße Stoffe, die sich nach dem entsprechenden oben erwähnten Verfahren herstellen lassen.

Die bei der Durchführung des erfindungsgemäßen Verfahrens zur Herstellung von Verbindungen der Formel (II) als Ausgangsstoffe benötigten Azolylmethyl-ketone der Formel (IV) sind bekannt oder lassen sich nach prinzipiell bekannten Verfahren in einfacher Weise herstellen (vergl. DE-4 2 431 407 und EP-A 0 353 558).

Die bei dem erfindungsgemäßen Verfahren zur Herstellung von Verbindungen der Formel (II) als Reaktionskomponenten benötigten Phosphonium-Salze der Formel (V) sind ebenfalls bekannt oder lassen sich nach prinzipiell bekannten Methoden herstellen. So erhalt man Phosphonium-Salze der Formel (V), indem man Benzylhalogenide der Formel 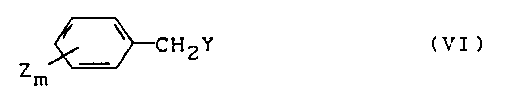 in welcher
Y, Z und m die oben angegebene Bedeutungen haben,
mit Triphenylphosphin gegebenenfalls in Gegenwart eines Verdünnungsmittels, wie Xylol oder Toluol, bei Temperaturen zwischen 110°C und 150°C umsetzt.

Als Basen kommen bei der Herstellung von Ethyl-triazolyl-Derivaten der Formel (II) nach dem obigen Verfahren alle für derartige Wittig-Reaktionen üblichen starken Säureakzeptoren in Betracht. Vorzugsweise verwendbar sind Alkoholate, wie Kalium-tert.-butylat, und Hydride, wie Natriumhydrid.

Als Verdünnungsmittel kommen bei dem obigen Verfahren zur Herstellung von Ethyl-triazolyl-Derivaten der Formel (II) alle für derartige Umsetzungen üblichen inerten organischen Solventien in Frage. Vorzugsweise verwendbar sind Ether, wie Diethylether, Dioxan oder Tetrahydrofuran, und ferner stark polare Lösungsmittel, wie Dimethylsulfoxid.

Die Reaktionstemperaturen können bei der Durchführung des obigen Verfahrens zur Herstellung von Ethyl-triazolyl-Derivaten der Formel (II) innerhalb eines bestimmten Bereiches variiert werden. Im allgemeinen arbeitet man bei Temperaturen zwischen -10°C und +80°C, vorzugsweise zwischen -5°C und +60°C.

Bei der Durchführung des obigen Verfahrens zur Herstellung von Ethyl-triazolyl-Derivaten der Formel (II) arbeitet man im allgemeinen unter Normaldruck. Es ist aber auch möglich, unter erhöhtem oder vermindertem Druck zu arbeiten.

Bei der Durchführung des obigen Verfahren zur Herstellung von Ethyl-triazolyl-Derivaten der Formel (II) setzt man auf 1 Mol an Azolylmethyl-keton der Formel (IV) im allgemeinen 1 bis 1,2 Mol an Phosphonium-Salz der Formel (V) sowie 1,2 bis 2 Mol an Base ein. Die Aufarbeitung erfolgt nach üblichen Methoden. Im allgemeinen geht man so vor, daß man das Reaktionsgemisch in Wasser oder eine gesättigte wäßrige Ammoniumchlorid-Lösung gießt, mit einem mit Wasser wenig mischbaren organischen Lösungsmittel extrahiert, die vereinigten organischen Phasen trocknet und unter vermindertem Druck einengt. Das verbleibende Produkt kann gegebenenfalls nach üblichen Methoden weiter gereinigt werden.

Bei der Durchführung des Verfahrens zur Herstellung von Ethyl-triazolyl-Derivaten der Formel (II) fallen die Verbindungen im allgemeinen in Form von E/Z-Isomerengemischen an. Die Isomerengemische lassen sich nach üblichen Methoden in die einzelnen geometrischen Isomeren auftrennen. Zur Isolierung einzelner Isomerer verfährt man im allgemeinen in der Weise, daß man die Isomerengemische einer säulenchromatographischen Trennung unterwirft.

Als Halogene der Formel (III) kommen bei der Durchführung des erfindungsgemäßen Verfahrens zur Herstellung von Verbindungen der Formel (I) Fluor, Chlor und Brom in Betracht, ferner gemischte Halogene, wie Chlor-(I)-fluorid, Brom-(I)-fluorid oder Brom-(I)-chlorid (vergl. Methodicum Chimicum, F. Korte, Bd. 7, S. 842 (1976)).

Als Verbindungen, die Halogene der Formel (III) liefern, können Sulfurylchlorid, N-Brom-succinimid mit Salzsäure, N-Chlorsuccinimid mit Bromwasserstoffsäure oder N-Chlorsuccinimid mit Fluorwasserstoff und Pyridin verwendet werden (vergl, Synthesis 1973, 780).

Die Addition von Halogenen an Ethyl-triazolyl-Derivate der Formel (II) kann durch Bestrahlung mit Licht, durch Einwirkung von Wärme, durch radikalbildende Substanzen, wie organische Peroxide, durch oberflächenaktive Stoffe, wie Aktivkohle, oder Metallsalze, wie Kupfer(II)-chlorid oder Eisen(III)-chlorid, begünstigt werden.

Als Verdünnungsmittel können bei der Durchführung des erfindungsgemäßen Verfahrens zur Herstellung von Verbindungen der Formel (I) alle für derartige Umsetzungen üblichen inerten Solventien eingesetzt werden. Vorzugsweise verwendbar sind halogenierte aliphatische Kohlenwasserstoffe, wie Methylenchlorid, Chloroform und Tetrachlorkohlenstoff, oder auch Phosphoroxychlorid.

Die Temperaturen können bei der Durchführung des erfindungsgemäßen Verfahrens zur Herstellung von Verbindungen der Formel (I) innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung des erfindungsgemäßen Verfahrens zur Herstellung von Verbindungen der Formel (I) arbeitet man im allgemeinen unter Normaldruck. Es ist aber auch möglich, unter erhöhtem Druck zu arbeiten,

Bei der Durchführung des erfindungsgemäßen Verfahrens zur Herstellung von Verbindungen der Formel (I) setzt man auf 1 Mol an Ethyl-triazolyl-Derivat der Formel (II) im allgemeinen eine äquivalente Menge oder auch einen Überschuß an Halogen der Formel (III) oder an Verbindungen, die Halogene der Formel (III) liefern, ein. Die Aufarbeitung erfolgt nach üblichen Methoden. Im allgemeinen geht man so vor, daß man das Reaktionsgemisch mit Wasser wäscht, die organische Phase trocknet und einengt. Es ist aber auch möglich, das Reaktionsgemisch nach beendeter Umsetzung direkt durch Abziehen der flüchtigen Komponenten unter vermindertem Druck einzuengen. Die anfallenden Substanzen können gegebenenfalls nach üblichen Methoden weiter gereinigt werden.

Die erfindungsgemäßen Ethyl-triazolyl-Derivate der Formeln (I) und (II) können in Säureadditions-Salze oder Metallsalz-Komplexe überführt werden.

Zur Herstellung von Säureadditions-Salzen -Salzen der Verbindungen der Formeln (I) und (II) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formeln (I) und (II) können in einfacher Weise nach üblichen Salzbildungsmethoden, z.B. durch Lösen einer Verbindung der Formel (I) oder (II) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formeln (I) und (II) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formeln (I) und (II) können in einfacher Weise nach üblichen Verfahren erhalten werden, so z.B. durch Lösen des Metallsalzes in Alkohol, z.B. Ethanol und Hinzufügen zu Verbindungen der Formeln (I) oder (II). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Leptosphaeria nodorum, Cochliobolus sativus, Pyrenophora teres, Pseudocercosporella herpotrichoides, Erysiphe und Fusarium-Arten. Außerdem zeigen die erfindungsgemäßen Stoffe eine sehr gute Wirkung gegen Venturia, Sphaerotheca und Botrytis. Sie besitzen außerdem eine breite in-vitro-Wirkung.

Die erfindungsgemäßen Stoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen. Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Ouarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nicht-ionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je kg Saatgut, vorzugsweise 0,01 bis 10 g benötigt. Bei der Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Stoffe geht aus den folgenden Beispielen hervor.

### Herstellungsbeispiele

### Beispiel 1

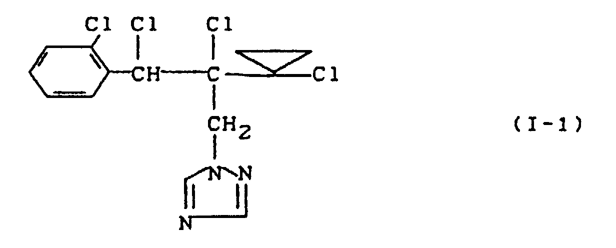

In eine Lösung von 20 g (68 mmol) 2-(1-Chlorcyclopropyl)-1-(1,2,4-triazol-1-yl)-3-(2-chlorphenyl)-2-propen (E/Z-Isomerengemisch) in 150 ml Dichlormethan wird bei 5°C bis 10°C unter Bestrahlung mit UV-Licht 2 Stunden lang ein Chlorgas-Strom eingeleitet. Das Reaktionsgemisch wird dann noch eine Stunde lang bei 5°C bis 10°C nachgerührt und anschließend mit Eiswasser gewaschen. Die organische Phase wird über Natriumsulfat getrocknet und durch Abziehen der flüchtigen Anteile unter vermindertem Druck eingeengt. Der verbleibende Rückstand wird mit einem Gemisch aus Cyclohexan:Ethylacetat = 5:1 als Laufmittel an Kieselgel chromatographiert. Man erhält auf diese Weise 9,4 g (38 % der Theorie) an 2-(1-Chlorcyclopropyl)-3-(2-chlorphenyl)-2,3-dichlor-1-(1,2,4-triazol-1-yl)-propan (Diastereomerengemisch) in Form einer Festsubstanz.
- 1H-NMR: (200 MHz, CDCl₃/TMS): δ = 0,3 - 1,3 (m,4H); 3,9 (d,1H); 5,25 (d,1H); 6,65 (s,1H); 7,2-7,4 (m,4H); 7,93 (s,1H); 8,22 (s,1H).

### Herstellung der Ausgangssubstanz

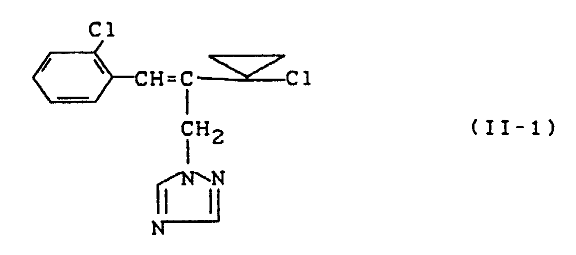

Eine Lösung von 115 g (271 mmol) 2-Chlorbenzyl-triphenylphosphonium-chlorid in 600 ml Tetrahydrofuran wird bei 0°C unter Rühren mit 36,6 g (326 mmol) Kalium-tert.-butylat versetzt. Nach beendeter Zugabe wird noch eine Stunde bei 40°C nachgerührt. Anschließend wird bei 30°C unter Rühren eine Lösung von 50,4 g (271 mmol) 1-Chlorcyclopropyl-(1,2,4-triazol-1-yl)-methylketon in 300 ml Tetrahydrofuran zugetropft. Das Reaktionsgemisch wird 16 Stunden bei Raumtemperatur gerührt und dann in gesättigte, wäßrige Ammoniumchlorid-Lösung eingegossen. Man trennt die Phasen, extrahiert die wäßrige Phase mehrfach mit Ethylacetat, trocknet die vereinigten organischen Phasen über Natriumsulfat und engt durch Abziehen des Lösungsmittels unter vermindertem Druck ein. Der verbleibende Rückstand wird mit einem Gemisch aus Cyclohexan:Ethylacetat = 2:1 als Laufmittel an Kieselgel chromatographiert. Man erhält auf diese Weise 61,5 g (77 % der Theorie) an 2-(1-Chlorcyclopropyl)-1-(1,2,4-triazol-1-yl)-3-(2-chlorphenyl)-2-propen (E/Z-Isomerengemisch) in Form einer Festsubstanz vom Schmelzpunkt 82-85°C.

### Beispiel 2

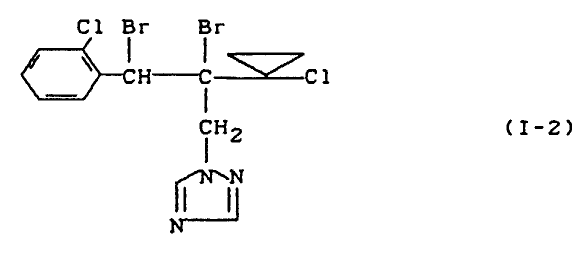

In eine Lösung von 4 g (13,6 mmol) 2-(1-Chlorcyclopropyl)-1-(1,2,4-triazol-1-yl)-3-(2-chlorphenyl)-2-propen (E/Z-Isomerengemisch) in 50 ml Dichlormethan werden unter Rückfluß und unter Bestrahlung mit UV-Licht 2,61 g (16,3 mmol) Brom eingetropft. Nach beendeter Zugabe wird noch 6 Stunden unter Bestrahlung mit UV-Licht unter Rückfluß erhitzt. Anschließend fügt man 0,9 ml Brom hinzu und kocht unter Bestrahlung mit UV-Licht weitere 16 Stunden unter Rückfluß. Danach wird das Reaktionsgemisch auf Raumtemperatur abgekühlt und durch Abziehen der flüchtigen Anteile unter vermindertem Druck eingeengt. Der verbleibende Rückstand wird mit einem Gemisch aus Cyclohexan:Ethylacetat = 5:1 als Laufmittel an Kieselgel chromatographiert. Man erhält auf diese Weise 0,97 g (16 % der Theorie) an 2-(1-Chlor-cyclopropyl)-3-(2-chlorphenyl)-2,3- dibrom-1-(1,2,4-triazol-1-yl)-propan (Diastereomerengemisch) in Form einer Festsubstanz.
- 1H-NMR: (200 MHz, CDCl₃/TMS): δ = 0,6 - 1,2 (m,4H); 4,0 (d,1H); 5,15 (d,1H); 6,63 (s,1H); 7,2-7,4 (m,4H); 7,95 (s,1H); 8,15 (s,1H).

Nach den zuvor angegebenen Methoden werden auch die in der folgenden Tabelle 2 aufgeführten Ethyl-triazolyl-Derivate der Formel (I) hergestellt,

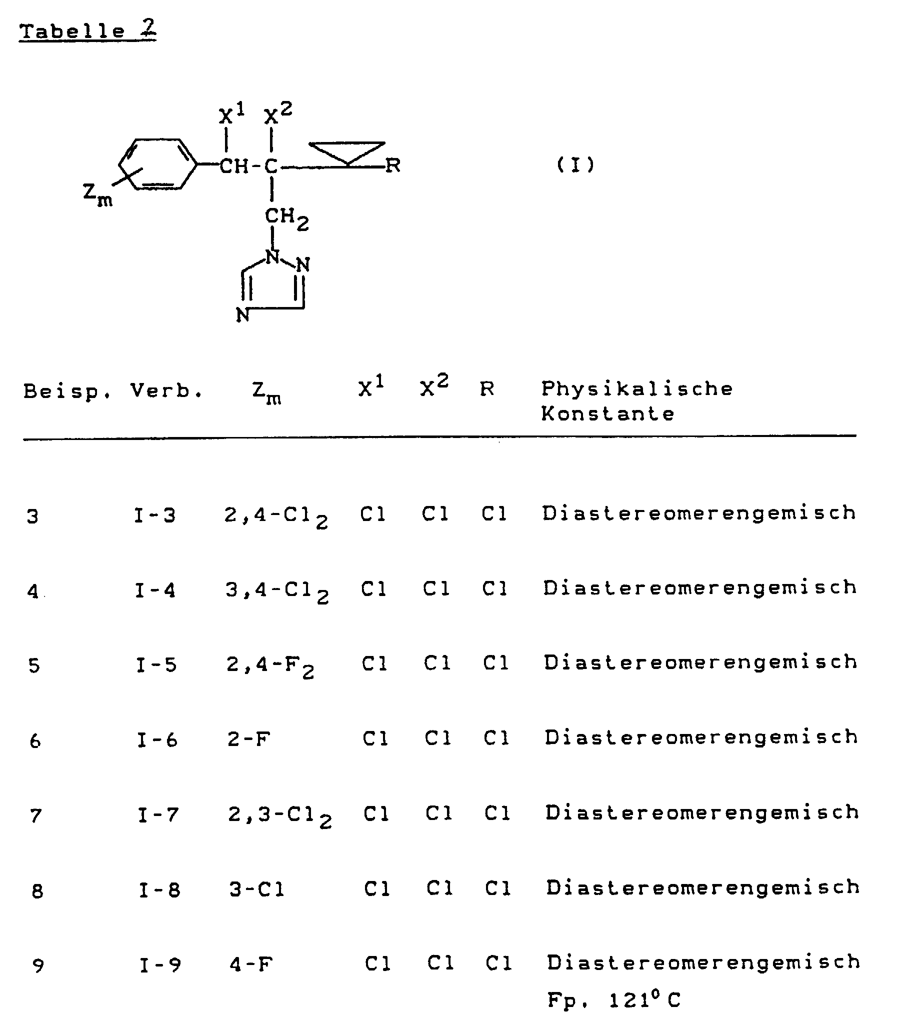

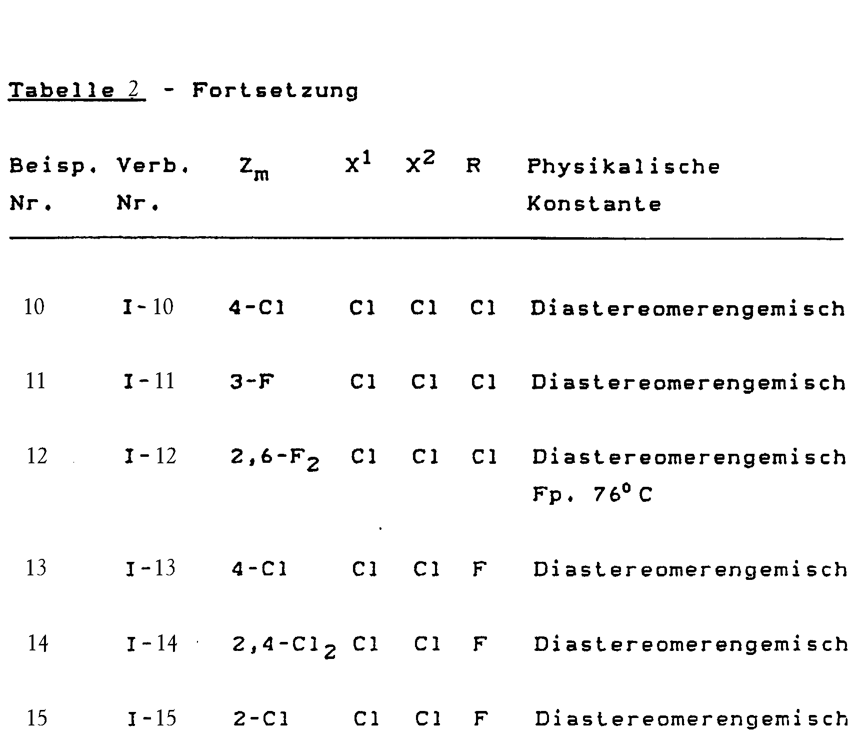

Zur Charakterisierung von Verbindungen aus Tabelle 2 dienen die folgenden ¹H-NMR-Signale (CDCl₃, TMS, 200 MHz, δ-Werte):
- Beispiel 3:: 0,2-0,8 (m, 2H), 1,1-1,6 (m, 2H), 3,8-4,1 (m, 1H), 5,1-5,4 (m, 1H), 6,6 (s, 1H), 7,3-7,6 (m, 3H), 8,0 und 8,1 (s, zus. 1H), 8,05 und 8,15 (s, zus. 1H)
- Beispiel 4:: 0,3-0,5 (m, 1H), 0,65-0,85 (m, 1H), 1,1-1,2 (m, 1H), 1,4-1,6 (m, 1H), 4,0 (d, 1H), 4,9 (d, 1H), 5,8 (s, 1H), 7,5 (m, 2H), 7,75 (d, 1H), 7,95 (s, 1H), 8,2 (s, 1H)
- Beispiel 5:: 0,1-0,8 (m, 2H), 1,1-1,6 (m, 2H), 3,9 (d), 4,9 (d), 5,1 (d, zus. 2H), 5,8 (s, 1H), 6,95-7,2 (m, 2H), 7,5-7,7 (m, 1H), 8,1 (s, 1H), 8,4 (s, 1H)
- Beispiel 6:: 0,2-0,8 (m, 2H), 1,1-1,6 (m, 2H), 4,0 (d), 5,1 (d), 5,15 (m, zus. 2H), 6,2 und 6,3 (s, zus. 1H), 7,0-7,5 (m, 3H), 7,8-8,0 (m, 1H), 7,95 und 8,0 (s, zus. 1H), 8,25 und 8,45 (s, zus. 1H)
- Beispiel 7:: 0,3-0,8 (m, 2H), 1,2-1,6 (m, 2H), 3,7-4,0 (m, 1H), 5,0-5,3 (m, 1H), 6,35 und 6,7 (s, zus. 1H), 7,1-7,6 (m, 2H), 7,9-8,1 (m, 1H), 7,95 und 8,05 (s, zus. 1H), 8,25 und 8,4 (s, zus. 1H)
- Beispiel 8:: 0,2-0,8 (m, 2H), 1,1-1,6 (m, 2H), 3,95 (d, 1H), 4,9 (d, 1H), 5,75 und 5,85 (s, zus. 1H), 7,2-7,7 (m, 4H), 7,95 und 8,0 (s. zus. 1H), 8,2 und 8,3 (s. zus. 1H)
- Beispiel 9:: 0,1-0,8 (m, 4H), 5,2 (d, 2H), 5,8 (s, 1H), 7,0-7,2 (m, 2H), 7,6-7,8 (m, 2H), 8,05 (s, 1H), 8,4 (s, 1H)
- Beispiel 10:: 0,3-0,5 (m, 1H), 0,6-0,8 (m, 1H), 1,1-1,2 (m, 1H), 1,4-1,6 (m, 1H), 3,95 (d, 1H), 4,9 (d, 1H), 5,85 (s, 1H), 7,4 (d, 2H), 7,6 (d, 2H), 7,95 (s, 1H), 8,25 (s, 1H)
- Beispiel 11:: 0,2-0,8 (m, 2H), 1,1-1,6 (m, 2H), 3,95 (d), 4,9 (d), 5,1 (d, zus. 2H), 5,75 und 5,85 (s, zus. 1H), 7,0-7,6 (m, 4H), 7,95 und 8,0 (s, zus. 1H, 8,25-8,3 (s, 1H)
- Beispiel 12:: 0,3-0,7 (m, 2H), 1,1-1,6 (m, 2H), 4,25 (d, 1H), 5,15 (d, 1H), 6,2 (s, 1H), 6,9-7,5 (m, 3H) 8,0 (s, 1H), 8,3 (s, 1H)
- Beispiel 13:: 0,2-0,5 (m, 1H), 0,6-0,9 (m, 1H), 1,0-1,4 (m, 2H), 4,2 (d, 1H), 4,8 (d, 1H), 5,6 (s, 1H), 7,4 (d, 2H), 7,6 (d, 2H), 8,0 (s, 1H), 8,2 (s, 1H)
- Beispiel 14:: 0,1-0,4 (m, 1H), 0,6-0,9 (m, 1H), 1,0-1,4 (m, 2H), 4,05 (d, 1H), 5,1 (d, 1H), 6,3 (s, 1H), 7,4-7,6 (m, 2H), 7,9-8,1 (m, 1H), 8,0 (s, 1H), 8,2 (s, 1H)
- Beispiel 15:: 0,3-1,4 (m, 4H), 4,15(d), 5,1 (d) und 5,2 (m, zus. 2H), 6,3 und 6,4 (s, zus. 1H), 7,2-7,4 (m, 4H), 8,1 (s) und 8,35 (s, zus. 1H), 8,3 und 8,6 (s, zus. 1H)

Nach der im Beispiel 1 angegebenen Methode werden auch die in der folgenden Tabelle 3 aufgeführten Ethyl-triazolyl-Derivate der Formel (II) hergestellt.

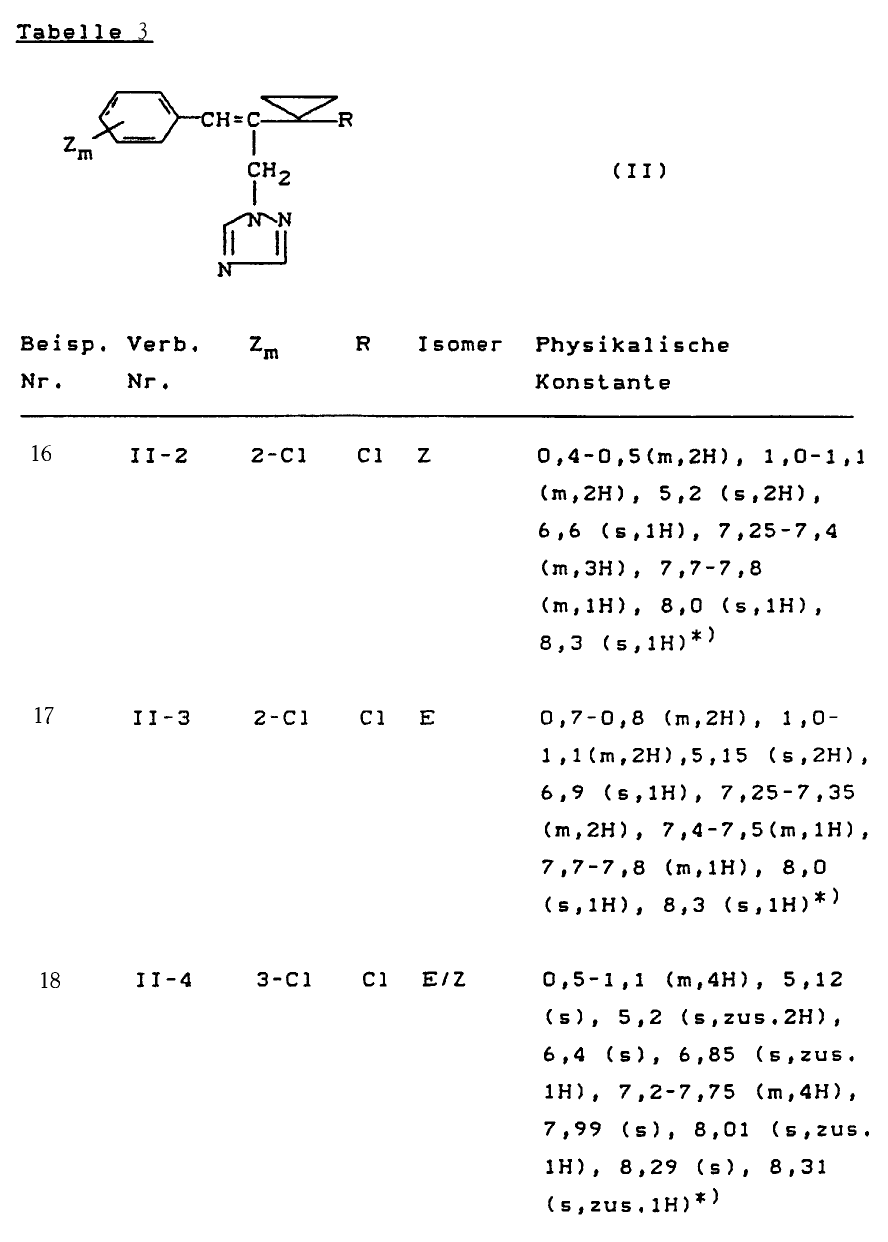

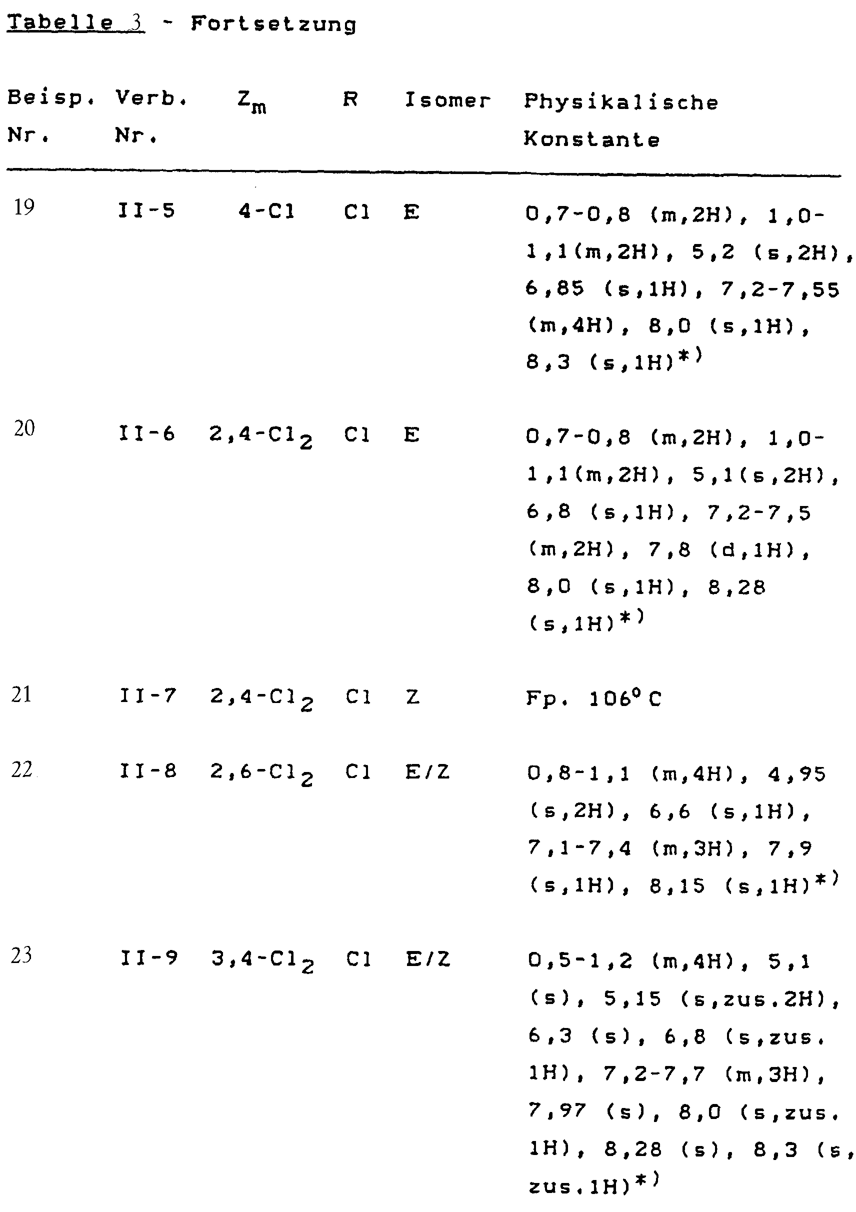

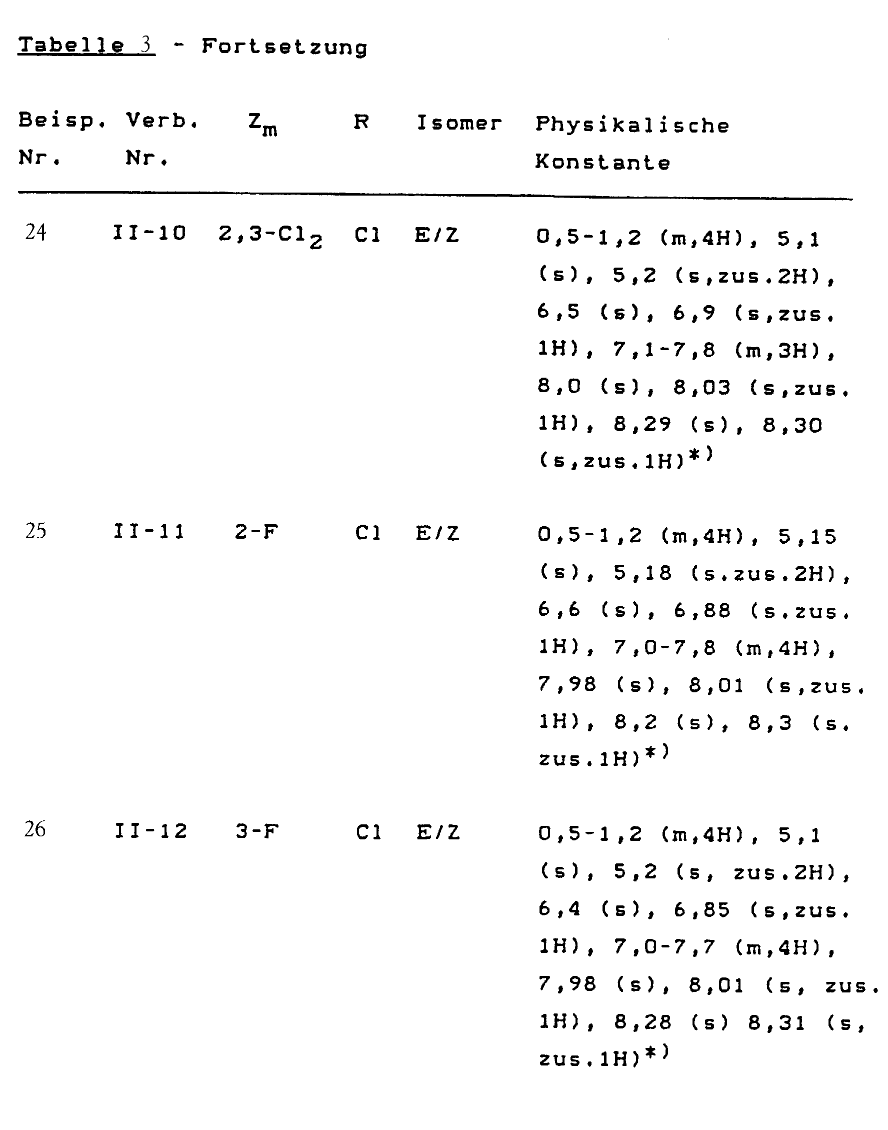

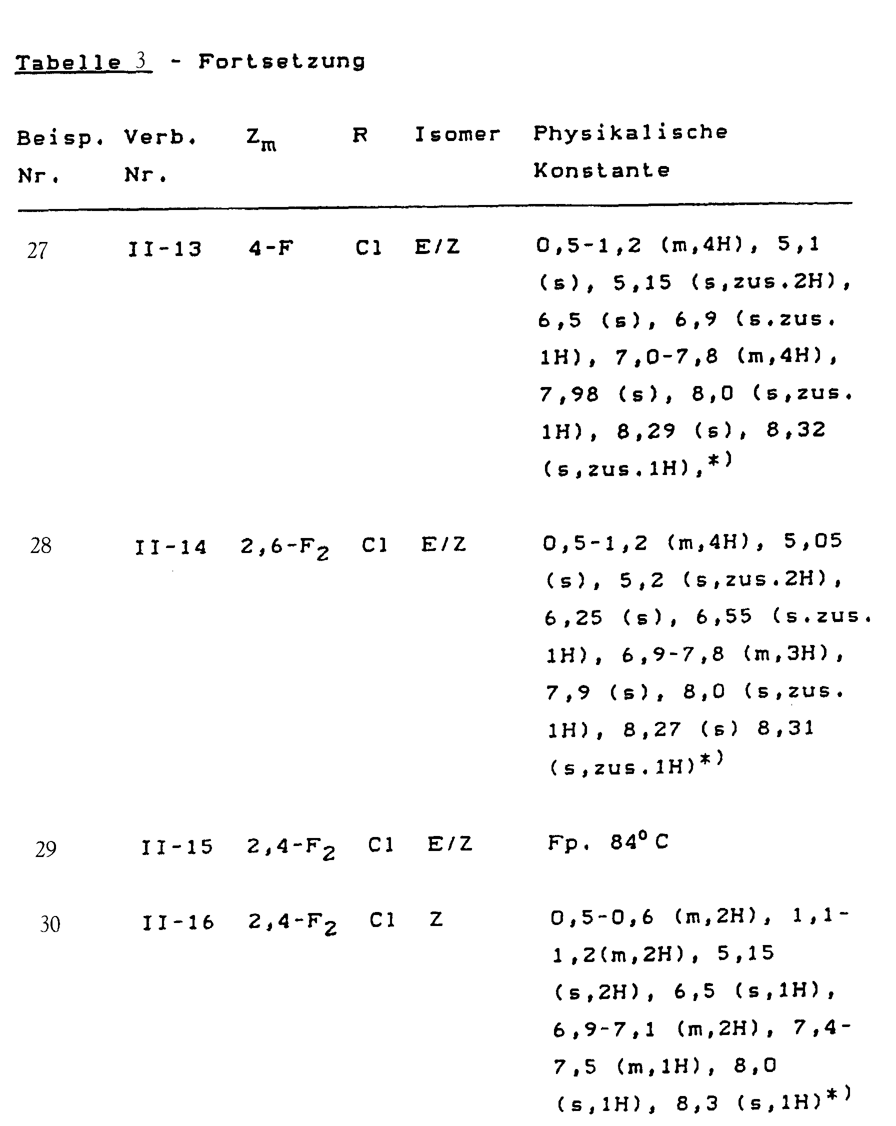

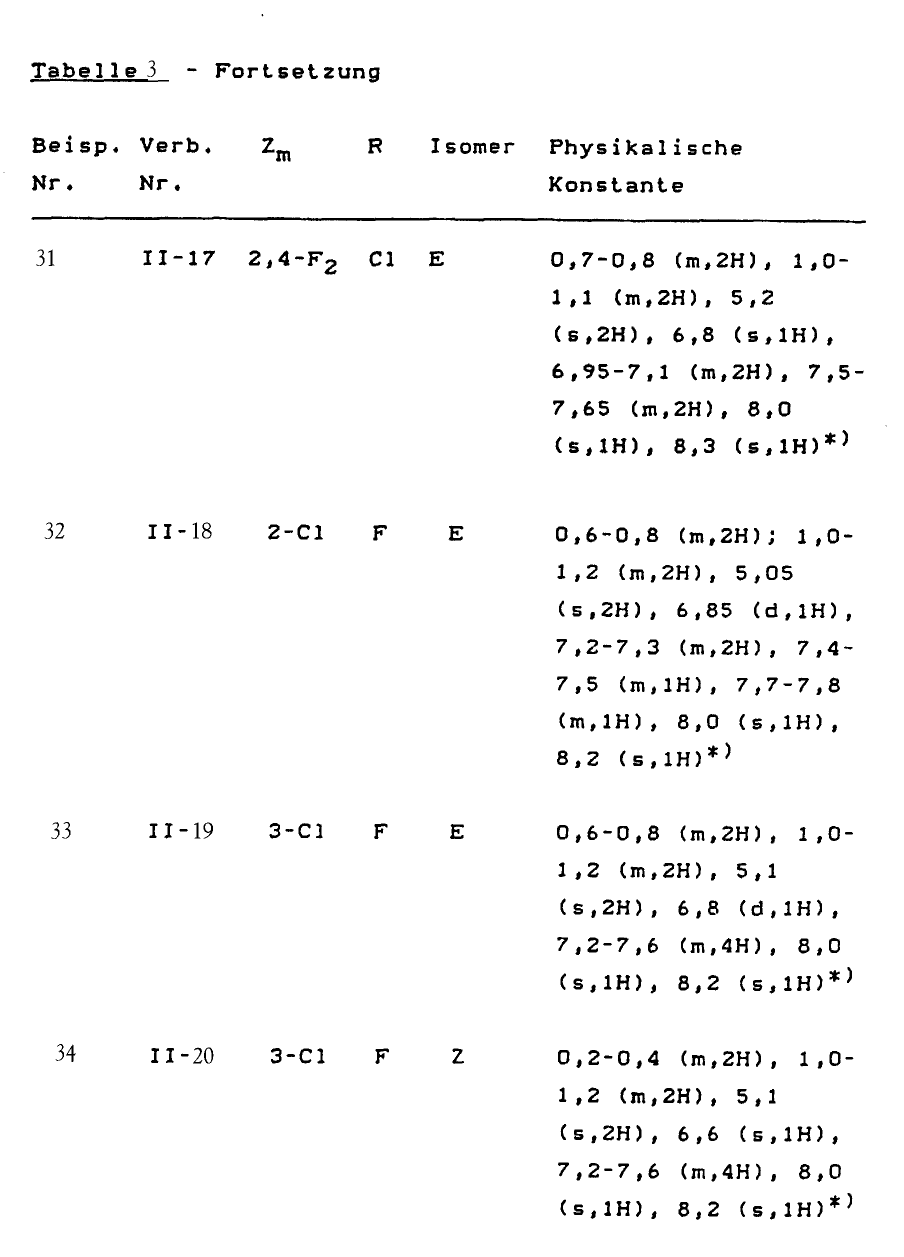

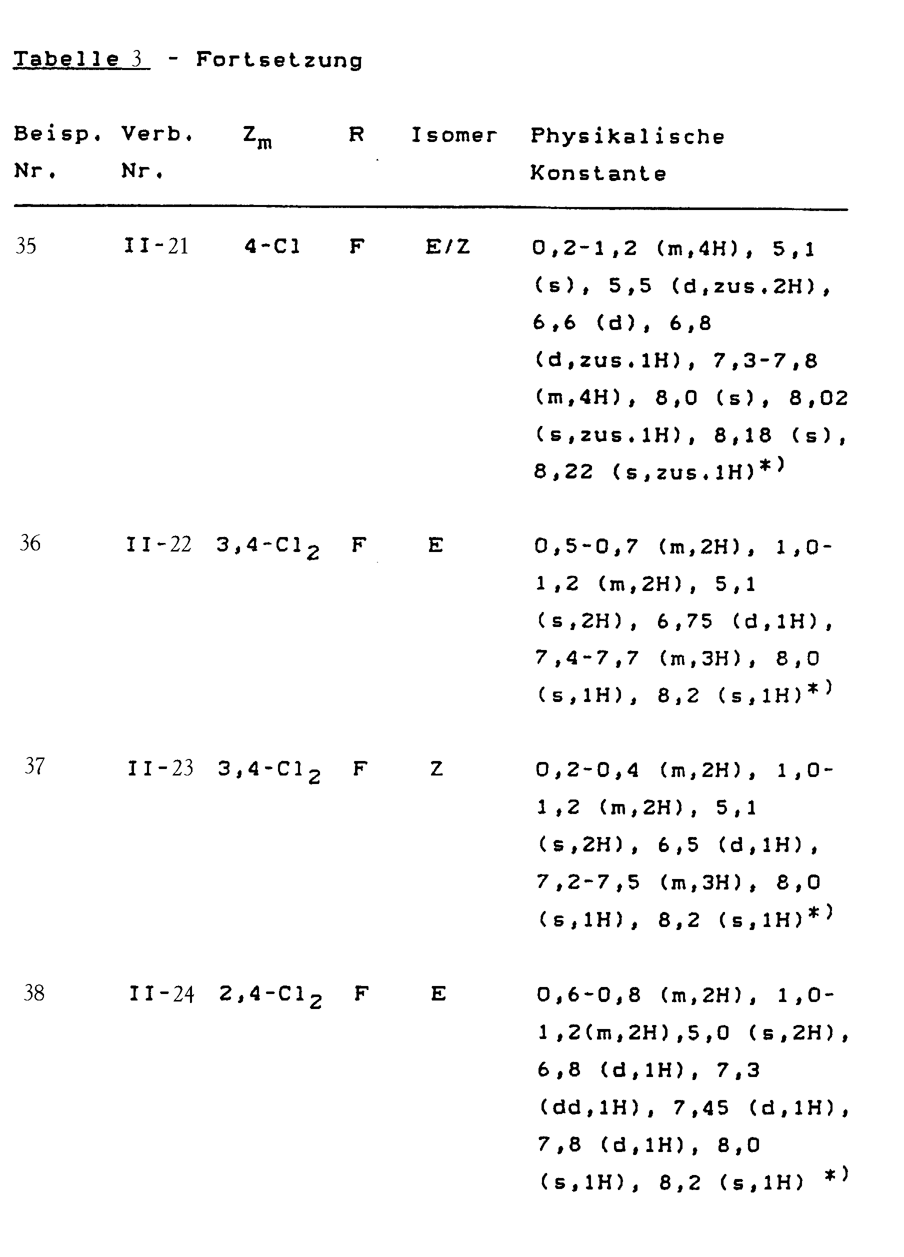

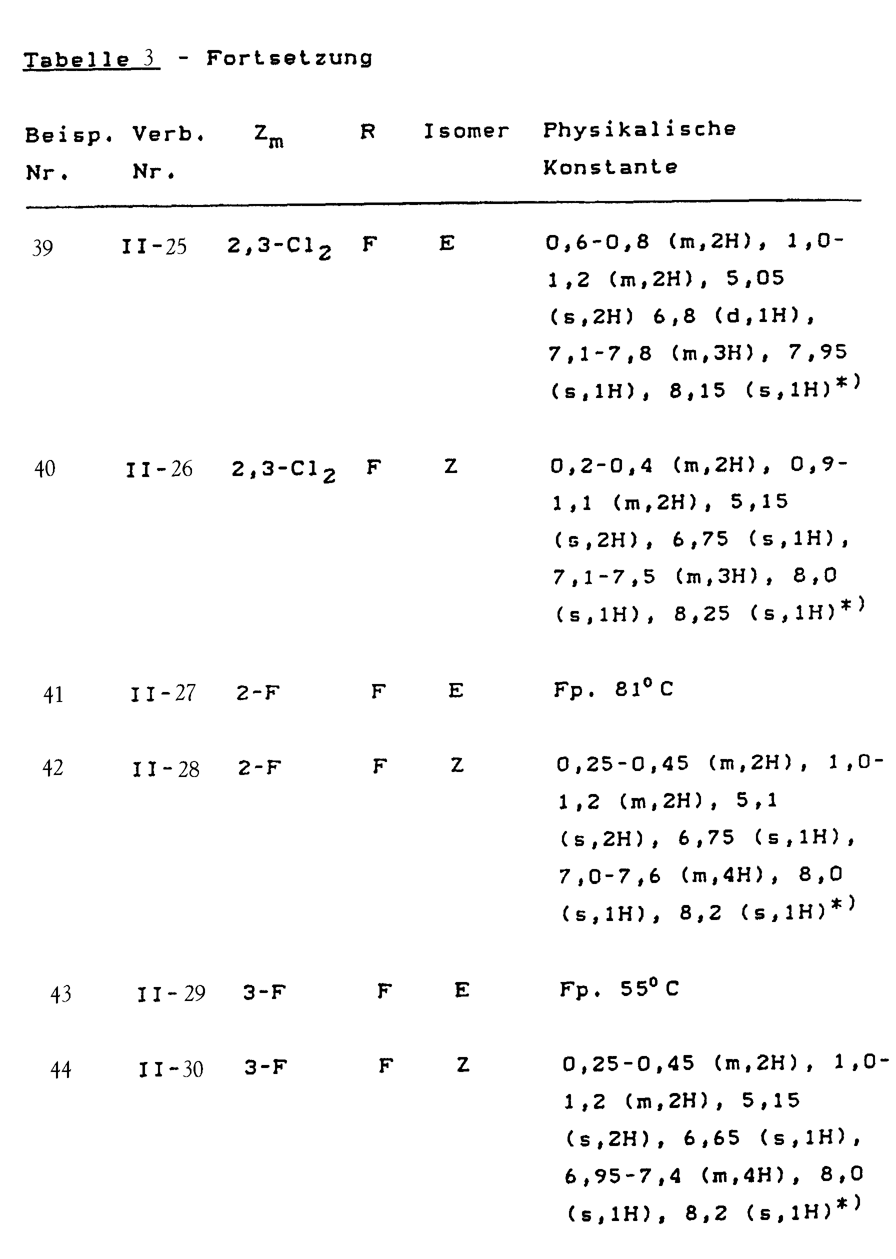

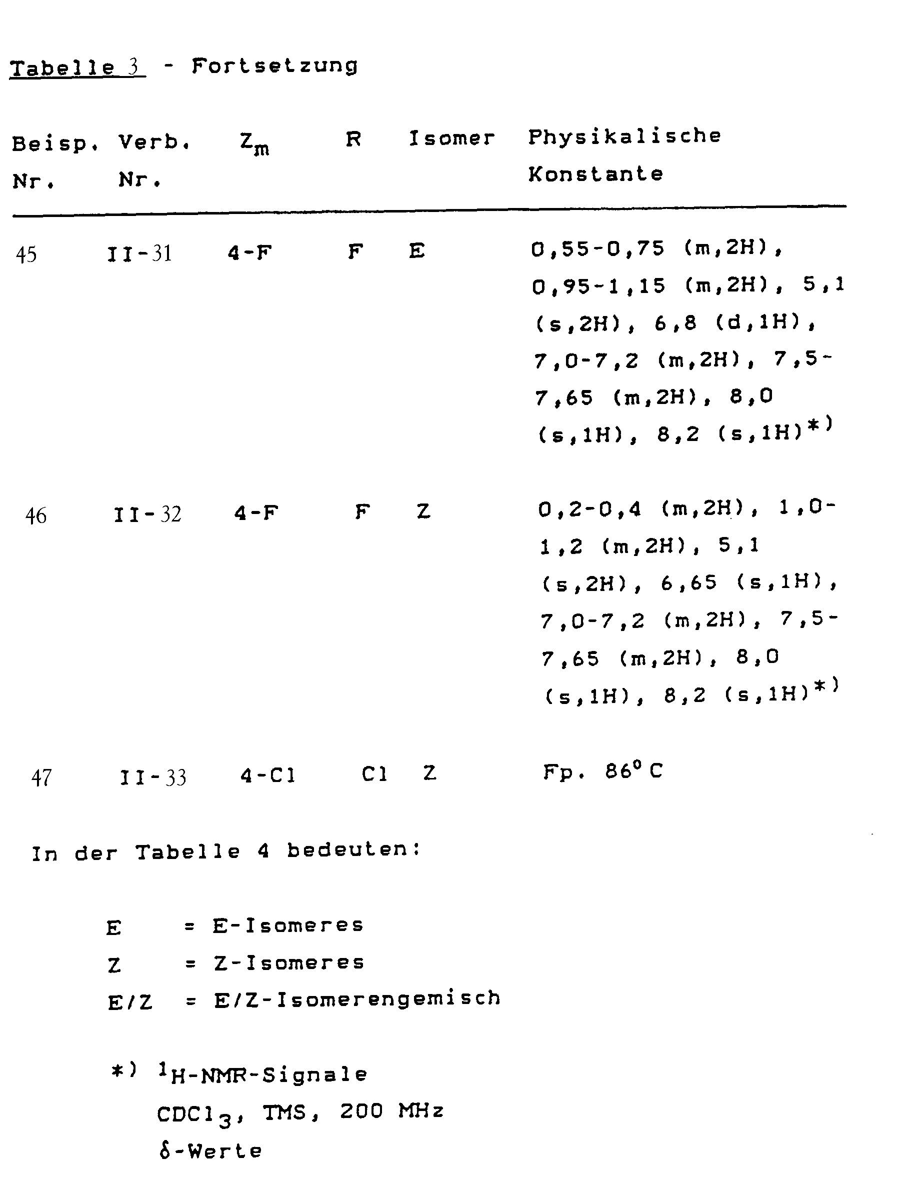

In den folgenden Verwendungsbeispielen wurden die Verbindungen der nachstehend aufgeführten Formeln als Vergleichssubstanzen eingesetzt.

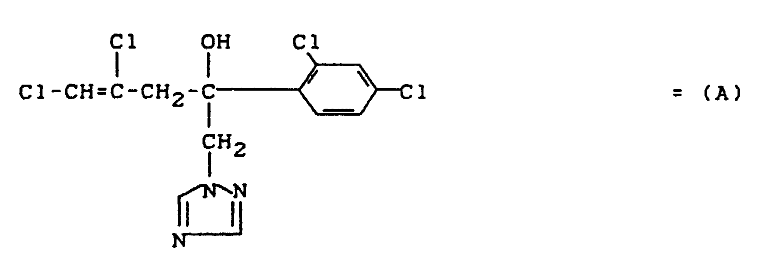

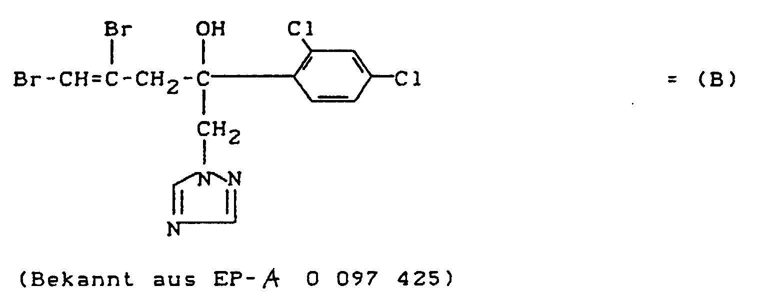

### Beispiel A

### Erysiphe-Test (Gerste) / protektiv

| | |
|---|---|
| Lösungsmittel | 100 Gew.-Teile Dimethylformamid |
| Emulgator | 0,25 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.
Die Pflanzen werden ein einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung der Formel (I-1) bei einer Konzentration von 25 ppm in der Spritzflüssigkeit eine Wirksamkeit von über 90 %, während der Wirkungsgrad für die Vergleichssubstanzen (A) und (B) zwischen 0 und 25 % liegt.

### Beispiel B

### Erysiphe-Test (Weizen) / protektiv

| | |
|---|---|
| Lösungsmittel | 100 Gew.-Teile Dimethylformamid |
| Emulgator | 0,25 Gew.-Teile Alkylarylpolyglykolether |

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung der Formel (I-1) bei einer Konzentration von 25 ppm in der Spritzflüssigkeit eine Wirksamkeit von fast 80 %, während der Wirkungsgrad für die Vergleichssubstanzen (A) und (B) zwischen 0 und 16 % liegt.

### Beispiel C

### Leptosphaeria nodorum-Test (Weizen) / protektiv

| | |
|---|---|
| Lösungsmittel | 100 Gew.-Teile Dimethylformamid |
| Emulgator | 0,25 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

In diesem Test zeigt die erfindungsgemäße Verbindung der Formel (I-1) bei einer Konzentration von 250 ppm in der Spritzflüssigkeit eine Wirksamkeit von 100 %, während der Wirkungsgrad der Vergleichssubstanz (A) 75 % beträgt.

### Beispiel D

### Pseudocercosporella herpotrichoides-Test (Weizen) / protektiv

| | |
|---|---|
| Lösungsmittel | 100 Gew.-Teile Dimethylformamid |
| Emulgator | 0,25 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

In diesem Test zeigen die erfindungsgemäßen Verbindungen der Formeln (I-1) und (I-2) bei einer Konzentration von 250 ppm in der Spritzflüssigkeit eine Wirksamkeit von 80 bis 100 %, während der Wirkungsgrad für die Vergleichssubstanzen (A) und (B) 50 % bzw. 0 % beträgt.

### Beispiel E

### Pyrenophora-teres-Test (Gerste) / protektiv

| | |
|---|---|
| Lösungsmittel | 100 Gew.-Teile Dimethylformamid |
| Emulgator | 0,25 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigt die erfindungsgemäße Verbindung der Formel (I-1) bei einer Konzentration von 250 ppm in der Spritzflüssigkeit einen Wirkungsgrad von 100 %.

### Beispiel F

### Uncinula-Test (Rebe) / protektiv

| | |
|---|---|
| Lösungsmittel | 4,7 Gew.-Teile Aceton |
| Emulgator | 0,2 Gew.-Teile Alkylarylpolyglykol-ether |

Zur Herstellung einer zweckmäßigen Wirkstoffzubereitung vermischt man 1 Gewichtsteil Wirkstoff mit den angegebenen Mengen Losungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden anschließend bei 23°C bis 24°C und bei einer relativen Luftfeuchtigkeit von ca. 75 % im Gewächshaus aufgestellt.

14 Tage nach der Inokulation erfolgt die Auswertung.

In diesem Test zeigen die erfindungsgemäßen Stoffe der Formeln (I-1) und (I-2) bei einer Konzentration von 10 ppm in der Spritzflüssigkeit einen Wirkungsgrad von 100 %.

### Beispiel G

### Erysiphe-Test (Gerste) / protektiv

| | |
|---|---|
| Lösungsmittel | 14,8 Gewichtsteile Dimethylformamid |
| Emulgator | 1,2 Gewichtsteile Alkylarylpolyglykol-ether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (II-1), (II-3) und (II-10) bei einer Aufwandmenge von 200 g/ha einen Wirkungsgrad von 100 %.

### Beispiel H

### Erysiphe-Test (Gerste) / protektiv

| | |
|---|---|
| Lösungsmittel | 100 Gewichtsteile Dimethylformamid |
| Emulgator | 0,25 Gewichsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wikrstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-1), (I-3) und (I-11) bei einer Konzentration von 250 ppm in der Spritzflüssigkeit einen Wirkungsgrad von 100 %.

### Beispiel I

### Podosphaera-Test (Apfel) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelschorferregers (Podosphaera leucotricha) inokuliert.

Die Pflanzen werden dann im Gewächshaus bei 23°C und einer relativen Luftfeuchtigkeit von ca. 70 % aufgestellt.

10 Tage nach der Inokulation wird der Wirkungsgrad ermittelt und in % ausgedrückt. Dabei bedeuten 0 %, daß keine Wirkung vorhanden ist (unbehandelte Kontrolle) und 100 %, daß kein Befall auftritt.

In diesem Test zeigen die erfindungsgemäßen Verbindungen (II-6), (II-23) und (II-26) bei einer Konzentration von 10 ppm in der Spritzflüssigkeit einen Wirkungsgrad von 100 %.

## Patentansprüche

1. Ethyl-triazolyl-Derivate der Formel 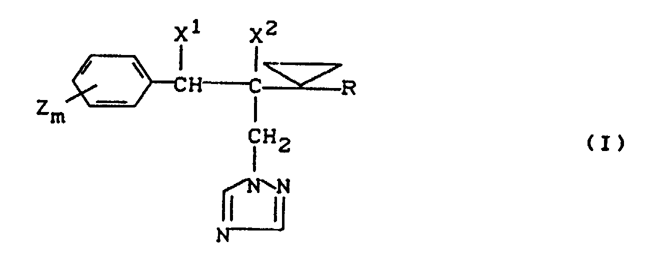 in welcher
R für Fluor oder Chlor steht,
X¹ für Fluor, Chlor oder Brom steht,
X² für Fluor, Chlor oder Brom steht,
Z für Fluor oder Chlor steht und
m für die Zahlen 0, 1 oder 2 steht,
sowie deren Säureadditions-Salze und Metallsalz-Komplexe.

2. Verfahren zur Herstellung von Ethyl-triazolyl-Derivaten der Formel (I) gemäß Anspruch 1 sowie von deren Säureadditions-Salzen und Metallsalz-Komplexen, dadurch gekennzeichnet, daß man Ethyl-triazolyl-Derivate der Formel 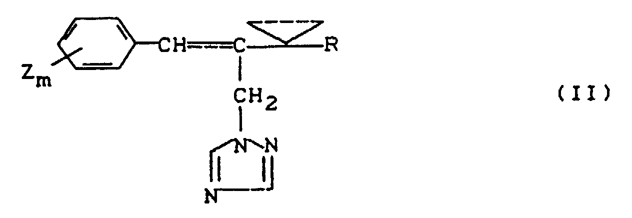 in welcher
R, Z und m die im Anspruch 1 angegebenen Bedeutungen haben,
mit Halogenen der Formel
X¹-X² (III)
in welcher
X¹ und X² die im Anspruch 1 angegebenen Bedeutungen haben,
oder mit Verbindungen, die Halogene der Formel (III) liefern, in Gegenwart eines Verdünnungsmittels und gegebenenfalls unter Bestrahlung umsetzt,
und gegebenenfalls an die so erhaltenen Verbindungen der Formel (I) eine Säure oder ein Metallsalz addiert.

3. Ethyl-triazolyl-Derivate der Formel 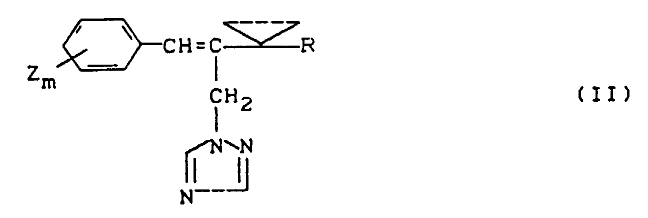 in welcher
R, Z und m die im Anspruch 1 angegebenen Bedeutungen haben,
sowie deren Säureadditions-Salze und Metallsalz-Komplexe.

4. Verfahren zur Herstellung von Ethyl-triazolyl- Derivaten der Formel (II) gemäß Anspruch 3 sowie von deren Säureadditions-Salzen und Metallsalz-Komplexen, dadurch gekennzeichnet, daß man Azolyl-methyl-ketone der Formel 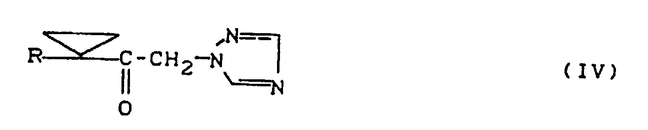 in welcher
R die im Anspruch 1 angegebene Bedeutung hat,
mit Phosphonium-Salzen der Formel 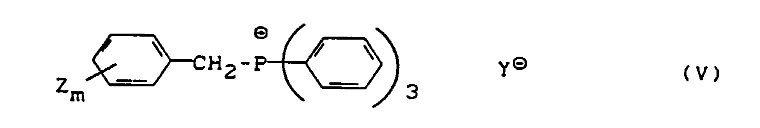 in welcher
Z und m die im Anspruch 1 angegebenen Bedeutungen haben und
Y für Chlor oder Brom steht,
in Gegenwart einer Base und in Gegenwart eines Verdünnungsmittels umsetzt,
und gegebenenfalls an die so erhaltenen Verbindungen der Formel (II) eine Säure oder Metallsalz addiert.

5. Fungizide Mittel, gekennzeichnet durch einen Gehalt an mindestens einem Ethyl-triazolyl-Derivat der Formel (I) oder (II) gemäß Ansprüchen 1 und 3 bzw. an einem Säureadditions-Salz oder Metallsalz-Komplex eines Ethyl-triazolyl-Derivates der Formel (I) oder (II).

6. Verwendung von Ethyl-triazolyl-Derivaten der Formeln (I) und (II) gemäß Ansprüchen 1 und 3 bzw. von deren Säureadditions-Salzen und Metallsalz-Komplexen zur Bekämpfung von phytopathogenen Pilzen.

7. Verfahren zur Bekämpfung von phytopathogenen Pilzen, dadurch gekennzeichnet, daß man Ethyl-triazolyl-Derivate der Formeln (I) und (II) gemäß Ansprüchen 1 und 3 bzw. deren Säureadditions-Salze oder Metallsalz-Komplexe auf die Pilze und/oder deren Lebensraum einwirken läßt.

8. Verfahren zur Herstellung von fungiziden Mitteln, dadurch gekennzeichnet, daß man Ethyl-triazolyl-Derivate der Formeln (I) und (II) gemäß Ansprüchen 1 und 3 bzw. deren Säureadditions-Salze oder Metallsalz-Komplexe mit Streckmitteln und/oder oberflächenaktiven Stoffen vermischt.

## Claims

1. Ethyl-triazolyl derivatives of the formula 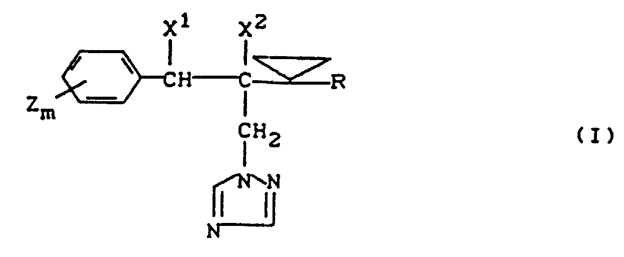 in which
R represents fluorine or chlorine,
X¹ represents fluorine, chlorine or bromine,
X² represents fluorine, chlorine or bromine,
Z represents fluorine or chlorine and
m represents the numbers 0, 1 or 2,
and their acid addition salts and metal salt complexes.

2. Process for the preparation of ethyl-triazolyl derivatives of the formula (I) according to Claim 1 and of their acid addition salts and metal salt complexes, characterized in that ethyl-triazolyl derivatives of the formula 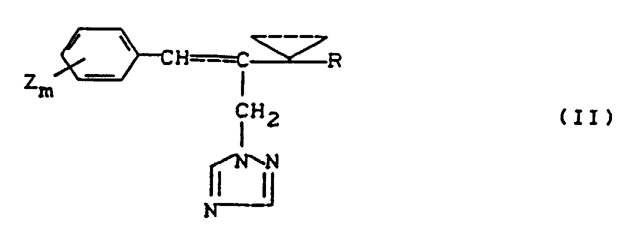 in which
R, Z and m have the meanings given in Claim 1
are reacted with halogens of the formula
X¹-X² (III)
in which
X¹ and X² have the meanings given in Claim 1
or with compounds which give halogens of the formula (III) in the presence of a diluent and, if appropriate, with irradiation,
and, if appropriate, the resulting compounds of the formula (I) are subjected to an addition reaction with an acid or a metal salt.

3. Ethyl-triazolyl derivatives of the formula 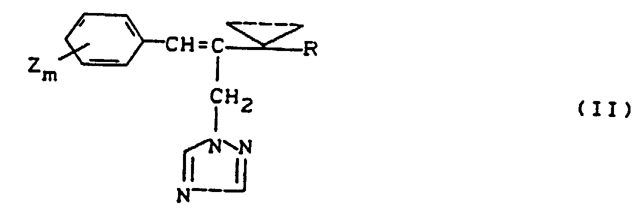 in which
R, Z and m have the meanings given in Claim 1,
and their acid addition salts or metal salt complexes.

4. Process for the preparation of ethyl-triazolyl derivatives of the formula (II) according to Claim 3
and of their acid addition salts and metal salt complexes, characterized in that azolyl-methyl ketones of the formula 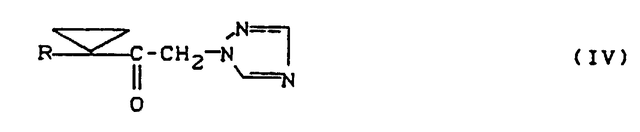 in which
R has the meaning given in Claim 1
are reacted with phosphonium salts of the formula 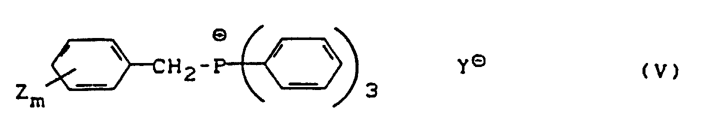 in which
Z and m have the meanings given in Claim 1
and
Y represents chlorine or bromine,
in the presence of a base and in the presence of a diluent,
and, if appropriate, the resulting compounds of the formula (II) are subjected to an addition reaction with an acid or metal salt.

5. Fungicidal compositions, characterized in that they comprise at least one ethyl-triazolyl derivative of the formula (I) or (II) according to Claims 1 and 3, or an acid addition salt or metal salt complex of an ethyl-triazolyl derivative of the formula (I) or (II).

6. Use of ethyl-triazolyl derivatives of the formula (I) and (II) according to Claims 1 and 3, or of their acid addition salts and metal salt complexes, for controlling phytopathogenic fungi.

7. Method of controlling phytopathogenic fungi, characterized in that ethyl-triazolyl derivatives of the formula (I) and (II) according to Claims 1 and 3, or their acid addition salts or metal salt complexes, are allowed to act on the fungi and/or their environment.

8. Process for the preparation of fungicidal compositions, characterized in that ethyl-triazolyl derivatives of the formula (I) and (II) according to Claims 1 and 3, or their acid addition salts or metal salt complexes, are mixed with extenders and/or surfactants.

## Revendications

1. Dérivés d'éthyl-triazolyle de formule 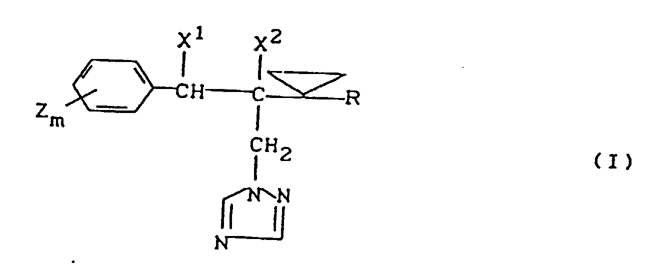 dans laquelle
R représente du fluor ou du chlore,
X¹ représente du fluor, du chlore ou du brome,
X² représente du fluor, du chlore ou du brome,
Z représente du fluor ou du chlore et
m représente les nombres 0, 1 ou 2,
ainsi que leurs sels d'addition d'acides et leurs complexes de sels métalliques.

2. Procédé de production de dérivés d'éthyl-triazolyle de formule (I) suivant la revendication 1
ainsi que de leurs sels d'addition d'acides et de leurs complexes de sels métalliques, caractérisé en ce qu'on fait réagir des dérivés d'éthyl-triazolyle de formule 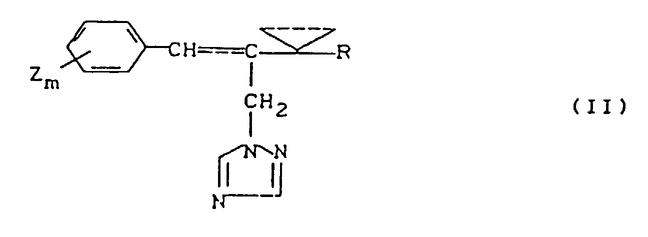 dans laquelle
R, Z et m ont les définitions indiquées dans la revendication 1,
avec des halogènes de formule
X¹-X² (III)
dans laquelle
X¹ et X² ont les définitions indiquées dans la revendication 1,
ou avec des composés qui fournissent les halogènes de la formule (III), en présence d'un diluant et, le cas échéant, sous irradiation,
et on additionne éventuellement sur les composés de formule (I) ainsi obtenus un acide ou un sel métallique.

3. Dérivés d'éthyl-triazolyle de formule 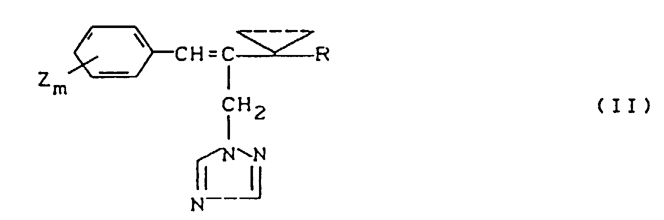 dans laquelle
R, Z et m ont les définitions indiquées dans la revendication 1,
ainsi que leurs sels d'addition d'acides et leurs complexes de sels métalliques.

4. Procédé de production de dérivés d'éthyl-triazolyle de formule (II) suivant la revendication 3
ainsi que de leurs sels d'addition d'acides et de leurs complexes de sels métalliques, caractérisé en ce qu'on fait réagir des azolyl-méthyl-cétones de formule 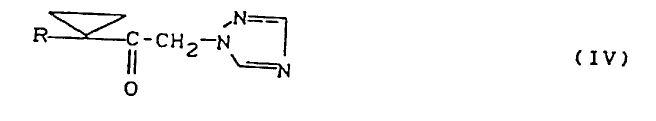 dans laquelle
R a la définition indiquée dans la revendication 1,
avec des sels de phosphonium de formule 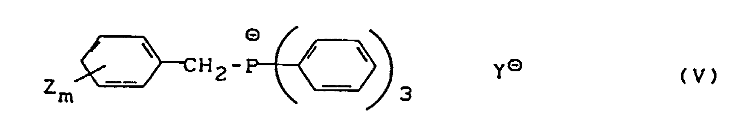 dans laquelle
Z et m ont les définitions indiquées dans la revendication 1,
et
Y représente du chlore ou du brome,
en présence d'une base et en présence d'un diluant,
et on additionne, le cas échéant, un acide ou un sel métallique sur les composés de formule (II) ainsi obtenus.

5. Compositions fongicides, caractérisées par une teneur en au moins un dérivé d'éthyl-triazolyle de formule (I) ou (II) suivant les revendications 1 et 3 ou en un sel d'addition d'acide ou un complexe de sel métallique d'un dérivé d'éthyl-triazolyle de formule (I) ou (II).

6. Utilisation de dérivés d'éthyl-triazolyle de formules (I) et (II) suivant les revendications 1 et 3 ou de leurs sels d'addition d'acides et de leurs complexes de sels métalliques pour combattre des champignons phytopathogènes.

7. Procédé pour combattre des champignons phytopathogènes, caractérisé en ce qu'on fait agir sur les champignons et/ou sur leur milieu des dérivés d'éthyl-triazolyle de formules (I) et (II) suivant les revendications 1 et 3 ou leurs sels d'addition d'acides ou leurs complexes de sels métalliques.

8. Procédé de préparation de compositions fongicides, caractérisé en ce qu'on mélange des dérivés d'éthyl-triazolyle de formules (I) et (II) suivant les revendications 1 et 3 ou leurs sels d'addition d'acides ou leurs complexes de sels métalliques avec des diluants et/ou des agents tensio-actifs.
